# EUROPÄISCHE PATENTSCHRIFT

(11) **EP 0 615 430 B1**
(45) Veröffentlichungstag und Bekanntmachung des Hinweises auf die Patenterteilung: **04.03.1998**
(21) Anmeldenummer: 93921861.6
(22) Anmeldetag: 28.09.1993
(51) Int. Cl.: A61F 2/74, A61F 2/64

(54) **SCHWUNGPHASENSTEUERVORRICHTUNG**
SWING PHASE CONTROL DEVICE
DISPOSITIF DE COMMANDE DE MOUVEMENTS OSCILLANTS EN PHASE

(30) Priorität: 02.10.1992 DE 4233247
(43) Veröffentlichungstag der Anmeldung: 21.09.1994
(73) Patentinhaber: BIEDERMANN MOTECH GmbH, D-78054 Villingen-Schwenningen (DE)
(72) Erfinder: FITZLAFF, Gerhard, D-78050 VS-Villingen (DE)
(74) Vertreter: Prüfer, Lutz H., Dipl.-Phys.
(86) Internationale Anmeldenummer: EP9302627
(87) Internationale Veröffentlichungsnummer: WO9407442

(56) Entgegenhaltungen:
- EP-A- 0 097 226
- EP-A- 0 560 652
- DE-B- 1 075 277
- FR-A- 1 565 589
- US-A- 4 212 087
- US-A- 5 092 902
- OELHYDRAULIK UND PNEUMATIK Bd. 8, Nr. 9 , September 1964 Seiten 352 - 357 J. ZICK 'DIE VERWENDUNG VON HYDRAULISCHEN ELEMENTEN IN PROTHESEN'

## Beschreibung

Die Erfindung bezieht sich auf eine Schwungphasensteuervorrichtung für ein künstliches Kniegelenk mit einer Kolbenzylindereinrichtung.

Bei einer derartigen Schwungphasensteuervorrichtung kann das Eintauchen des Kolbens der Kolbenzylindereinrichtung durch die erste Drossel und damit das Beugen des künstlichen Kniegelenkes gesteuert werden. Wenn das Kniegelenk wieder gestreckt werden soll, muß die Person, die das künstliche Kniegelenk benutzt, den Unterschenkel wieder nach vorne schwingen und in die Streckung mit dem Oberschenkel bringen. Hierbei tritt die gleiche Drosselwirkung wie bei der Beugung auf. Da jedoch der Beugungsvorgang und der Streckungsvorgang getrennte Vorgänge sind, führt eine gleiche Drosselwirkung zu einem unnatürlichen Bewegungsablauf. Zwar ist die Drossel einstellbar und kann damit an den jeweiligen Laufstil angepaßt werden, jedoch weist sie keine dynamische Anpassung an unterschiedliche Bewegungsabläufe der Person auf. Die Drossel wirkt also nur Optimal entweder für langsame oder für schnelle Bewegungen.

In der EP-A-0 560 562, die Stand der Technik nach Art. 54(3) EPÜ bildet, ist eine Schwungphasensteuervorrichtung für ein künstliches Kniegelenk mit einer Kolbenzylindereinrichtung beschrieben, die eine erste Kammer auf einer Seite des Kolbens und eine zweite Kammer auf der gegenüberliegenden Seite des Kolbens aufweist, wobei die beiden Kammern über eine erste Drossel miteinander verbindbar sind. Eine zweite Drossel ist zum Verbinden der zweiten Kammer mit einem Fluidreservat vorgesehen, wobei eine Einrichtung so ausgebildet ist, daß sie bei Erreichen bzw. Überschreiten eines vorbestimmten Druckes in der zweiten Kammer die zweite Drossel schließt.

Es ist daher Aufgabe der Erfindung, eine Schwungphasensteuervorrichtung der eingangs beschriebenen Art zu schaffen, die einen natürlichen Bewegungsablauf ermöglicht und die insbesondere für unterschiedlich schnelle Bewegungsabläufe geeignet ist.

Diese Aufgabe wird gelöst durch eine Schwungphasensteuervorrichtung der eingangs beschriebenen Art, die die Merkmale des Patentanspruches 1 aufweist.

Durch das Vorsehen der zweiten Drossel ist es möglich, unterschiedliche Steuerungen für das Beugen und das Strecken des Kniegelenkes vorzusehen. Der Bewegungsablauf wirkt natürlicher. Die Drossel wird so eingestellt, daß sie für langsame und mittelschnelle Bewegungen geeignet ist. Wenn die Person sich aber sehr schnell bewegt, taucht der Kolben auch sehr schnell in den Zylinder ein und komprimiert das Fluid in der zweiten Kammer. Hat der Druck des Fluids in der zweiten Kammer den vorbestimmten Wert überschritten, wird die zweite Drossel geschlossen. Das Fluid kann nicht mehr durch diese zweite Drossel austreten. Dabei baut sich der Druck in der zweiten Kammer rascher auf und führt wie eine Luftfeder zu einem dynamischen Zurückstoßen des Kolbens in dem Zylinder. Das Kniegelenk wird wieder schnell und schwungvoll gestreckt. Dadurch ist durch die erfindungsgemäße Schwungphasensteuervorrichtung auch ein schwungvolles schnelles Gehen mit der Prothese möglich.

Bevorzugte Ausgestaltungen der erfindungsgemäßen Schwungphasensteuervorrichtung ergeben sich aus den Unteransprüchen.

Weitere Merkmale und Zweckmäßigkeiten der erfindungsgemäßen Schwungphasensteuervorrichtung ergeben sich aus der Beschreibung eines Ausführungsbeispieles anhand der begleitenden Figur. In der begleitenden Figur ist eine Schnittansicht einer Ausführungsform der Schwungphasensteuervorrichtung gezeigt.

Die Schwungphasensteuervorrichtung 1 weist eine Zylinderkolbeneinrichtung 2 mit einem Zylinder 3 und einem Kolben 12 auf. Der Zylinder 3 umfaßt einen rohrförmigen Abschnitt 5 mit einem Deckelteil 4 an seinem einen Ende und einem Bodenteil 6 an dem gegenüberliegenden Ende.

Das Deckelteil 4 weist eine Einlaßöffnung 7 auf, die eine Verbindung zwischen der Umgebungsluft und einer in dem Zylinder 3 gebildeten eingangsseitigen ersten Kammer 8 ermöglicht. Die Einlaßöffnung 7 ist mit einem Rückschlagventil 9 versehen. Das Rückschlagventil 9 ist als Flatterventil ausgebildet, welches sich zu der ersten Kammer 8 hin öffnet. Konzentrisch zu dem Zylinder 3 ist eine Bohrung 10 in einer Buchse in dem Deckelteil 4 vorgesehen. In der Bohrung 10 befindet sich ein Gleitlager für eine Kolbenstange 11, die mit dem Kolben 12 der Zylinderkolbeneinrichtung 2 verbunden ist. Auf der Außenseite der Einlaßöffnung 7 ist ein Filter 13 zum Schutz gegen das Eindringen unerwünschter Bestandteile aus der Außenluft vorgesehen. Zum Abdichten des Raumes zwischen der konzentrischen Bohrung 10 und der Kolbenstange 11 ist eine Ringdichtung 14 vorgesehen.

Das Deckelteil 4 ist auf den rohrförmigen Abschnitt 5 aufgeschraubt. Eine Ringdichtung 15 dichtet die Verbindung zwischen dem rohrförmigen Abschnitt 5 und dem Deckelteil 4 ab. Der rohrförmige Abschnitt 5 ist auf das Bodenteil 6 aufgeschraubt. Es ist eine Ringdichtung 16 zwischen dem rohrförmigen Abschnitt 5 und dem Bodenteil 6 zum Abdichten vorgesehen.

Auf der der ersten Kammer 8 abgewandten Seite des Kolbens 12 ist eine zweite Kammer 17 in dem rohrförmigen Abschnitt 5 gebildet. Auf der dem Kolben 12 entgegengesetzten Seite ist die zweite Kammer von dem Bodenteil 6 begrenzt.

In dem Bodenteil 6 ist konzentrisch eine in die zweite Kammer 17 mündende Bohrung 18 vorgesehen. Das Bodenteil 6 weist eine radiale Auslaßbohrung 19 auf, die in die Bohrung 18 mündet. Die Auslaßbohrung 19 ist durch ein Staubfilter 20 abgedeckt.

Auf der der zweiten Kammer 17 zugewandten Seite ist in der Bohrung 18 ein Steuerkolben 27 vorgesehen. Dieser Steuerkolben 27 weist eine ringförmige Dichtung 28, die an die Innenseite der konzentrischen Bohrung 18 anliegt, auf. Der Steuerkolben 27 weist eine konzentrische Bohrung 29 auf. In diese mündet nahe an ihrem der zweiten Kammer 17 zugewandten Rand eine Drosselbohrung, die eine Verbindung zwischen der Bohrung 29 und der Bohrung 18 bildet. Zur Begrenzung der Bewegung des Steuerkolbens 27 zur zweiten Kammer 17 hin ist eine mit dem Bodenteil verschraubte Anschlagplatte 25 vorgesehen. Der Steuerkolben 27 wird durch eine Druckfeder 30 gegen die Anschlagplatte 25 vorgespannt. Die Bohrung 18 und die Auslaßbohrung 19 bilden eine dritte Kammer 31. In dem an die Bohrung 18 angrenzenden eigentlichen Boden ist eine konzentrische Bohrung mit einem Gewinde 21 vorgesehen. In das Gewinde 21 ist eine Drosselstange 24 mit einem Gewindeabschnitt 22 und einem Rändelkopf 23 eingeschraubt. Die Drosselstange 24 ist nur soweit in die Bohrung 29 eingeschraubt, daß der Eingang der Drosselbohrung 32 in der vorgespannten Endstellung frei ist und erst nach einem vorbestimmten Weg des Steuerkolbens abgesperrt wird. Der Ausgang der Drosselbohrung 32 mündet in die dritte Kammer 31.

Die zweite Kammer 17 ist direkt mit der dritten Kammer 31 durch einen Durchgang 33 verbunden. Der Durchgang 33 weist ein Rückschlagventil 34 auf, das als Flatterventil ausgebildet ist. Das Rückschlagventil 34 öffnet sich zu der zweiten Kammer 17 hin.

Die Anschlagplatte weist Durchbrechungen 26 derart auf, daß die der zweiten Kammer zugewandte Oberfläche des Steuerkolbens 27 mit dem Druck in der zweiten Kammer beaufschlagt wird.

Der Kolben 12 weist eine konzentrische Bohrung 35 auf, die in die zweite Kammer 17 mündet. An einer der zweiten Kammer 17 abgewandten Seite steht die konzentrische Bohrung 35 über eine Querbohrung 36 mit der ersten Kammer in Verbindung. Die konzentrische Bohrung 35 weist ein sich zur zweiten Kammer hin öffnende Rückschlagventil 37 auf, das als Flatterventil ausgebildet ist. Die konzentrische Bohrung 35 weist einen sich zur ersten Kammer 8 hin konisch erweiternden Abschnitt 38 auf. Der Kolben 12 weist eine Ringdichtung 39 auf, die an den rohrförmigen Abschnitt 5 anliegt.

Die konzentrische Bohrung 35 des Kolbens 12 setzt sich in der mit dem Kolben an der dem Deckelteil 4 zugewandten Seite verbundenen Kolbenstange 11 fort, die Kolbenstange 11 ist also hohl ausgebildet. In der Kolbenstange 11 ist eine Steuerstange 40 vorgesehen. Die Steuerstange 40 weist ein Rändelteil 41 auf und ist über ein Gewinde 42 mit der Kolbenstange 11 verbunden. An ihrem dem Deckelteil 4 abgewandten Ende weist die Steuerstange 40 einen konischen Abschnitt 43 auf, der mit dem konischen Abschnitt 38 der konzentrischen Bohrung 35 zusammenwirkt. Durch Drehen am Rändelteil 41 kann die Steuerstange 40 in den konischen Abschnitt 38 bis zu einer Schließstellung hineingedreht oder aus ihm herausgedreht werden, so daß eine einstellbare Drossel gebildet ist.

Bei der Benutzung in einem Prothesen-Kniegelenk wird die Schwungphasensteuervorrichtung 1 mit einem mit dem freien Ende der Kolbenstange 11 verbundenen Befestigungsteil 44 mit dem Prothesenoberschenkelteil verbunden. Der Zylinder wird in seinem Bodenbereich mit einem weiteren Befestigungsteil 45 mit dem Prothesenunterschenkelteil verbunden.

Im folgenden soll der Betrieb der Schwungphasensteuervorrichtung beschrieben werden. Wenn das Kniegelenk gestreckt ist (Extension), befindet sich der Kolben 12 in seiner zurückgezogenen oberen, dem Deckelteil 4 benachbarten Stellung. Die erste Kammer 8 hat ihre minimale Größe. Die zweite Kammer 17 hat ihre maximale Größe. Wenn das künstliche Bein der Person, die die Schwungphasensteuervorrichtung benutzt, gebeugt wird, knickt das Kniegelenk ein (Flexion). Der Kolben 12 bewegt sich von seiner oberen in die untere Stellung. Umgebungsluft strömt durch das Rückschlagventil 9 in die sich vergrößernde erste Kammer 8 nach. Die Feder 30 und der Durchmesser der Drosselbohrung 32 sind so bemessen, daß bei normaler Betätigung der Druck in der zweiten Kammer 17 den Steuerkolben 27 noch nicht nach unten drücken kann. Daher liegt die Mündung des Drosselkanales 32 in der Bohrung 29 frei. Der nach unten gehende Kolben 12 verdrängt die in der zweiten Kammer 17 vorhandene Luft durch die Drosselbohrung 32. Durch die dritte Kammer 31 und die Auslaßöffnung 19 tritt die Luft dann nach außen aus.

Wenn der Unterschenkel der mit der Schwunghasensteuervorrichtung versehenen Prothese nach vorne geschwungen wird, streckt sich das Kniegelenk wieder. Der Kolben 12 geht in dem Zylinder 3 wieder nach oben. Das Rückschlagventil 9 ist in diesem Fall in geschlossener Stellung. Damit die Luft aus der ersten Kammer 8 in die zweite Kammer 17 fließen kann, ist die Steuerstange 40 so eingestellt, daß ein Durchlaßspalt an dem konischen Abschnitt 38 besteht. Durch diesen Spalt kann die Luft von der ersten in die zweite Kammer strömen, da sich das Rückschlagventil 37 öffnet. Die Spaltbreite und damit die Größe der durch den Spalt gebildete Drossel kann durch Einstellen des Rändelteiles 41 von der die Schwungphasensteuervorrichtung benutzenden Person eingestellt werden. Daher ist eine gute Anpassung an den normalen Bewegungsablauf möglich. Damit in der Kammer 17 kein Unterdruck entsteht, kann Luft durch die Auslaßöffnung 19, das Rückschlagventil 34 und den Durchgang 33 in die zweite Kammer 17 nachströmen.

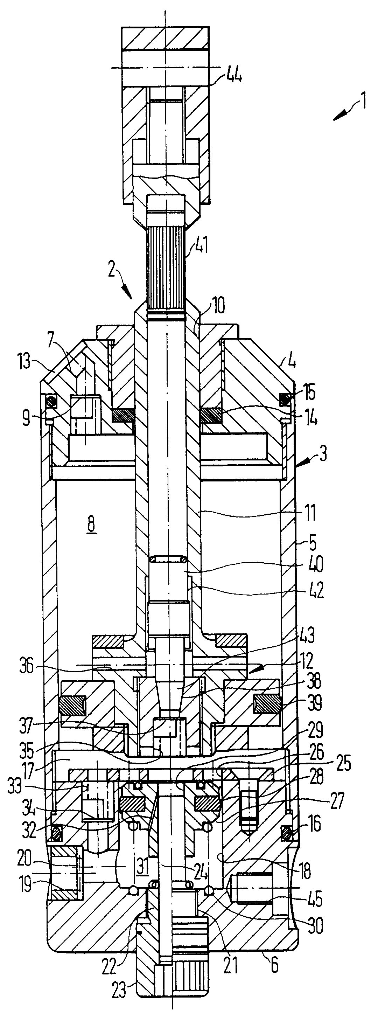

Wenn sich dagegen die die Schwungphasensteuervorrichtung benutzende Person schnell bewegt und dabei das Kniegelenk beugt, bewegt sich der Kolben 12 schnell in die in der Figur gezeigte Stellung. Dabei baut sich in der zweiten Kammer 17 schnell ein hoher Druck auf, da die Luft durch die Drosselbohrung 32 nicht schnell genug abfließen kann. Falls die Beugebewegung lange genug anhält, steigt der Druck in der zweiten Kammer 17 so hoch an, daß der durch die Öffnungen 26 auf die Oberfläche des Steuerkolbens 27 wirkende Druck ausreicht, um den Steuerkolben 27 gegen die Wirkung der Druckfeder 30 zur Bodenseite hin zu bewegen. Aufgrund dieser Bewegung beginnt die Drosselstange 24 die Drosselbohrung 32 abzudecken. Dadurch steigt der Druck in der zweiten Kammer 17 noch rascher an, der Steuerkolben 27 wird noch schneller zur Bodenseite gedrückt. Schließlich ist die Drosselbohrung 32 ganz abgedeckt. Dann wirkt die zweite Kammer 17 als Luftfeder, da durch den Spalt im Kolben 12 keine Luft abfließen kann, denn das Flatterventil 37 ist geschlossen.

Die durch die zweite Kammer 17 gebildete Luftfeder bewirkt, daß die eintauchende Bewegung des Kolbens 12 in dem Zylinder 3 umgekehrt wird und der Kolben 12 wieder aus dem Zylinder 3 herausgeschoben wird. Da die Aufwärtsbewegung des Kolbens 12 eine Streckung des Kniegelenkes bedeutet, wird also die Streckbewegung des Kniegelenkes durch das Umkehren der Bewegung des Kolbens 12, das durch die Luftfeder in der zweiten Kammer 17 verursacht wird, aktiv unterstützt. Also wird bei einer schnellen Beugebewegung auch eine schnelle Streckungsbewegung ermöglicht. Insgesamt kann sich die Person daher sehr viel schneller und auch sicherer bewegen. Eine Verzögerung der Streckung des Unterschenkels bei schnellen Bewegungen tritt also nicht auf.

Der Zeitpunkt der durch die Luftfeder induzierten Umkehrbewegung kann durch Einstellen der Drosselstange 24 mit Hilfe der Rändelschraube 22, 23 gesteuert werden und an die individuellen Gegebenheiten angepaßt werden.

Bei der oben beschriebenen Ausführungsform ist die Einlaßöffnung 7 der ersten Kammer 8 mit der Umgebungsluft verbunden. Desgleichen führt die Auslaßöffnung 19 der dritten Kammer 31 zur Umgebungsluft. Es handelt sich um ein offenes System, bei dem als Medium Luft benutzt wird. Gemäß einer anderen Ausführungsform ist ein geschlossenes System vorgesehen. Dabei wird die Schwungphasensteuervorrichtung von einem Außengehäuse umgeben. Dieses Außengehäuse ist hermetisch abgeschlossen. In einem solchen Fall ist das durch den Kolben 12 bewegte Medium nicht auf Luft beschränkt. Es sind andere Gase möglich, die insbesondere eine andere Dichte aufweisen und daher die Kennlinie der Schwungphasensteuervorrichtung beeinflussen. Für sehr harte Anwendungen kann auch ein Hydraulikmedium benutzt werden. Dann wird als geeignete Flüssigkeit Öl eingesetzt. In dem letzteren Fall ist die Umkehrbewegung allerdings sehr hart, da die Wirkung der Luft-"Feder" fehlt. Dieses kann insbesondere bei Sporteinsätzen angezeigt sein.

Wenn ein abgeschlossenes äußeres Gehäuse vorgesehen wird, besteht die Möglichkeit, daß außerhalb des Zylinders 3 ein Über- oder Unterdruck entsteht, da durch die Ansaugöffnung 7 zuviel von dem Medium angesaugt wird oder bei der Auswärtsbewegung zuviel von dem Medium ausgestoßen wird. Es wird dann über eine Membran ein Druckausgleich mit der Atmosphäre vorgesehen.

## Patentansprüche

1. Schwungphasensteuervorrichtung (1) für ein künstliches Kniegelenk mit einer Kolbenzylindereinrichtung (2) mit einer ersten Kammer (8) auf der einen Seite des Kolbens (12) und einer zweiten Kammer (17) auf der gegenüberliegenden Seite des Kolbens (12), die über eine in dem Kolben (12) angeordnete einstellbare erste Drossel (38, 43) miteinander verbindbar sind,
wobei die erste Kammer (8) mit einem Fluidreservoir verbindbar ist,
und wobei eine auf der dem Kolben (12) abgewandten Ausgangsseite der zweiten Kammer (17) angeordnete zweite Drossel (32) zum Verbinden der zweiten Kammer (17) mit dem Fluidreservoir
und eine Einrichtung, die so ausgebildet ist, daß sie bei Erreichen bzw. Überschreiten eines vorbestimmten Druckes in der zweiten Kammer (17) die zweite Drossel (32) schließt, vorgesehen sind.

2. Schwungphasensteuervorrichtung (1) nach Anspruch 1,
dadurch gekennzeichnet, daß die Einrichtung als ein Steuerkolben (27) ausgebildet ist, der entlang eines Weges hin und her bewegbar und in die Richtung zur zweiten Kammer (17) vorgespannt ist.

3. Schwungphasensteuervorrichtung (1) nach Anspruch 2,
dadurch gekennzeichnet, daß eine Drosselstange (24) vorgesehen ist und der Steuerkolben (27) eine der Drosselstange (24) entsprechende Bohrung (29) aufweist und auf der Drosselstange (24) geführt ist, daß der Steuerkolben (27) eine von einem Medium in der zweiten Kammer (17) beaufschlagte Fläche aufweist und daß die zweite Drossel (32) eingangsseitig so in die Bohrung (29) mündet, daß die Öffnung bei dem Überschreiten des vorbestimmten Druckes von der Drosselstange (24) geschlossen wird.

4. Schwungphasensteuervorrichtung (1) nach einem der Ansprüche 1 bis 3,
dadurch gekennzeichnet, daß zwischen der zweiten Kammer (17) und dem Fluidreservoir eine dritte Kammer (31) vorgesehen ist, in die der Drosselausgang mündet und die ausgangsseitig über einen Filter (20) mit dem Fluidreservoir verbunden ist.

5. Schwungphasensteuervorrichtung (1) nach Anspruch 4,
dadurch gekennzeichnet, daß die zweite Kammer (17) und die dritte Kammer (31) über ein sich zur zweiten Kammer (17) öffnendes Rückschlagventil (34) verbunden sind.

6. Schwungphasensteuervorrichtung (1) nach einem der Ansprüche 3 bis 5,
dadurch gekennzeichnet, daß die Relativstellung der Drosselstange (24) und der Bohrung (29) des Steuerkolbens (27) durch eine Einstellschraube (23) und ein Gewinde (22) einstellbar sind.

7. Schwungphasensteuervorrichtung (1) nach einem der Ansprüche 1 bis 6,
dadurch gekennzeichnet, daß die erste Kammer (8) einen Einlaß (7) mit einem sich zu der ersten Kammer (8) öffnenden dritten Rückschlagventil (9) aufweist.

8. Schwungphasensteuervorrichtung (1) nach einem der Ansprüche 1 bis 7,
dadurch gekennzeichnet, daß eine Bohrung (35) vorgesehen ist, die die erste (8) mit der zweiten (17) Kammer verbindet und einen konischen Abschnitt (38) aufweist,
daß der Kolben (12) mit einer Kolbenstange (11) verbunden ist, die hohl ist,
daß in der Kolbenstange (11) eine Steuerstange (40) mit einem konisch verlaufenden Ende (43) vorgesehen ist, und
daß das konisch verlaufende Ende (43) der Steuerstange (40) zum Einstellen der ersten Drossel in dem konischen Abschnitt (38) verschiebbar ist.

9. Schwungphasensteuervorrichtung (1) nach einem der Ansprüche 1 bis 8,
dadurch gekennzeichnet, daß der Kolben (12) mit einem Prothesenoberschenkelteil verbindbar ist,
daß der Zylinder (3) der Kolbenzylindereinrichtung (2) mit einem Prothesenunterschenkelteil verbindbar ist und
daß der Kolben (12) beim Beugen des Kniegelenkes in den Zylinder (3) eintaucht.

10. Schwungphasensteuervorrichtung (1) nach einem der Ansprüche 1 bis 9,
dadurch gekennzeichnet, daß das Fluid Luft ist und das Fluidreservoir aus der Umgebungsluft gebildet ist.

11. Schwungphasensteuervorrichtung (1) nach einem der Ansprüche 1 bis 9,
dadurch gekennzeichnet, daß das Fluid ein Hydraulikmedium oder ein Pneumatikmedium ist und daß das Fluidreservoir mit der Umgebung in Druckausgleich steht.

## Claims

1. Swing phase control device (1) for an artificial knee joint having a piston and cylinder arrangement (2) with a first chamber (8) on one side of the piston (12) and a second chamber (17) on the opposite side of the piston (12), which chambers are interconnectable via an adjustable first throttle (38, 43) arranged in the piston (12), the first chamber (8) being connectable to a fluid reservoir, and there being provided a second throttle (32), arranged on the outlet side of the second chamber (17) facing away from the piston (12), for connecting the second chamber (17) to the fluid reservoir, and a means which is designed so that it closes the second throttle (32) if a predetermined pressure is reached or exceeded in the second chamber (17).

2. Swing phase control device (1) according to Claim 1, characterized in that the means is designed as a control piston (27) which is movable to and fro along a path and is biased towards the second chamber (17).

3. Swing phase control device (1) according to Claim 2, characterized in that a throttle rod (24) is provided and the control piston (27) comprises a bore (29) corresponding to the throttle rod (24) and is guided on the throttle rod (24), in that the control piston (27) comprises a surface acted upon by a medium in the second chamber (17), and in that the second throttle (32) opens into the bore (29) on the inlet side so that the opening is closed by the throttle rod (24) if the predetermined pressure is exceeded.

4. Swing phase control device (1) according to one of Claims 1 to 3, characterized in that there is provided between the second chamber (17) and the fluid reservoir a third chamber (31), into which the throttle outlet opens and which is connected on the outlet side to the fluid reservoir via a filter (20).

5. Swing phase control device (1) according to Claim 4, characterized in that the second chamber (17) and the third chamber (31) are connected via a check valve (34) opening towards the second chamber (17).

6. Swing phase control device (1) according to one of Claims 3 to 5, characterized in that the relative position of the throttle rod (24) and the bore (29) of the control piston (27) is adjustable by an adjusting screw (23) and a thread (22).

7. Swing phase control device (1) according to one of Claims 1 to 6, characterized in that the first chamber (8) comprises an inlet (7) having a third check valve (9) opening towards the first chamber (8).

8. Swing phase control device (1) according to one of Claims 1 to 7, characterized in that there is provided a bore (35) which connects the first chamber (8) to the second chamber (17) and comprises a conical portion (38), in that the piston (12) is connected to a piston rod (11) which is hollow, in that a control rod (40) having a conical end (43) is provided in the piston rod (11), and in that the conical end (43) of the control rod (40) is displaceable in the conical portion (38) for adjusting the first throttle.

9. Swing phase control device (1) according to one of Claims 1 to 8, characterized in that the piston (12) is connectable to an upper leg part of a prosthesis, in that the cylinder (3) of the piston and cylinder arrangement (2) is connectable to a lower leg part of a prosthesis, and in that the piston (12) plunges into the cylinder (3) when the knee joint is flexed.

10. Swing phase control device (1) according to one of Claims 1 to 9, characterized in that the fluid is air and the fluid reservoir is formed from the ambient air.

11. Swing phase control device (1) according to one of Claims 1 to 9, characterized in that the fluid is a hydraulic medium or a pneumatic medium, and in that the fluid reservoir is in a state of pressure balance with the surroundings.

## Revendications

1. Dispositif de commande de mouvements oscillants en phase (1) pour une articulation du genou artificielle comprenant un organe à piston et cylindre (2), présentant une première chambre (1) sur une face du piston (12) et une deuxième chambre (17) sur la face opposée du piston (12), ces chambres étant susceptibles d'être reliées ensemble par l'intermédiaire d'un premier étranglement (38, 43) réglable, dispose dans le piston (12), la première chambre (8) étant susceptible d'être reliée à un réservoir de fluide et dans lequel sont prévus d'une part un deuxième étranglement (32), disposé sur la face de sortie, opposée au piston (12), de la deuxième chambre (17), pour assurer la liaison de la deuxième chambre (17) avec le réservoir de fluide, et d'autre part un organe, réalisé de telle manière que, en cas d'atteinte ou de dépassement d'une pression prédéterminée dans la deuxième chambre (17), le deuxième étranglement (32) soit fermé.

2. Dispositif de commande de mouvements oscillants en phase (1) selon la revendication 1, caractérisé en ce que l'organe est réalisé sous la forme d'un piston de commande (27) qui est déplacable alternativement le long d'un trajet, et est pré-contraint dans la direction de la deuxième chambre (17).

3. Dispositif de commande de mouvements oscillants en phase (1) selon la revendication 2, caractérisé en ce qu'est prévue une tige d'étranglement (24) et en ce que le piston de commande (27) comporte un alésage (29) correspondant à la tige d'étranglement (24) et est guidé sur la tige d'étranglement (24), et ce que le piston de commande (27) présente une surface sollicitée par un fluide se trouvant dans la deuxième chambre (17), et en ce que le deuxième étranglement (32) débouche, du côté entrée, dans l'alésage (29), de manière que l'ouverture soit fermée fermée par la tige de piston (24), en cas de dépassement de la pression prédéterminée.

4. Dispositif de commande de mouvements oscillants en phase (1) selon l'une des revendications 1 à 3, caractérisé en ce qu'entre la deuxième chambre (17) et le réservoir de fluide est prévue une troisième chambre (31), dans laquelle débouche la sortie d'étranglement et qui est reliée, du côté sortie, par un filtre (20), au réservoir de fluide.

5. Dispositif de commande de mouvements oscillants en phase (1) selon la revendication 4, caractérisé en ce que la deuxième chambre (17) et la troisième chambre (31) sont reliés par l'intermédiaire d'un clapet anti-retour (34) s'ouvrant vers la deuxième chambre (17).

6. Dispositif de commande de mouvements oscillants en phase (1) selon l'une des revendications 3 à 5, caractérisé en ce que la position relative de la tige d'étranglement (24) et l'alésage (29) du piston de commande (27) sont réglables au moyen d'une vis de réglage (23) et d'un filetage (22).

7. Dispositif de commande de mouvements oscillants en phase (1) selon l'une des revendications 1 à 6, caractérisé en ce la première chambre (8) présente une admission (7) avec un troisième clapet anti-retour (9) s'ouvrant vers la première chambre (8).

8. Dispositif de commande de mouvements oscillants en phase (1) selon l'une des revendications 1 à 7, caractérisé en ce qu'est prévu un alésage (35) qui relie la première (8) et la deuxième (17) chambres et présente un tronçon conique (38), en ce que le piston (12) est relié à une tige de piston (11) qui est creuse, en ce que dans la tige de piston (11) est prévue une tige de commande (40) ayant une extrémité (43) d'allure conique, et en ce que l'extrémité (43) d'allure conique de la tige de piston (40) est susceptible de coulisser pour assurer le réglage du premier organe d'étranglement dans le tronçon conique (38).

9. Dispositif de commande de mouvements oscillants en phase (1) selon l'une des revendications 1 à 8, caractérisé en ce que le piston (12) est susceptible d'être relié à une partie supérieure d'articulation d'une prothèse, en ce que le cylindre (3) de l'organe à piston et cylindre (2) est susceptible d'être relié à une partie inférieure d'articulation d'une prothèse, et en ce que le piston (12) rentre dans le cylindre (3) lorsque de l'articulation de genou ou de coude se plie.

10. Dispositif de commande de mouvements oscillants en phase (1) selon l'une des revendications 1 à 9, caractérisé en ce le fluide est de l'air et en ce que le réservoir de fluide est constitué par l'air ambiant.

11. Dispositif de commande de mouvements oscillants en phase (1) selon l'une des revendications 1 à 9, caractérise en ce le fluide est un fluide hydraulique ou un fluide pneumatique, et en ce que le réservoir de fluide est relié à l'ambiance en équilibre de pression.
